Europäisches Patentamt

European Patent Office

Office européen des brevets

(11) Publication number: **0 209 277**
**B1**

# EUROPEAN PATENT SPECIFICATION

(45) Date of publication of patent specification: **11.04.90**

(21) Application number: **86304939.1**

(22) Date of filing: **25.06.86**

(51) Int. Cl.⁵: **C 12 P 19/06,** C 09 K 7/02,
E 21 B 43/22 // C12R1:64

(54) Heteropolysaccharide and its production and use.

(30) Priority: **28.06.85 US 750704**

(43) Date of publication of application:
**21.01.87 Bulletin 87/04**

(45) Publication of the grant of the patent:
**11.04.90 Bulletin 90/15**

(84) Designated Contracting States:
**AT BE CH DE FR GB IT LI LU NL SE**

(56) References cited:
EP-A-0 012 552
EP-A-0 077 680
EP-A-0 130 647
US-A-4 352 741

CARBOHYDRATE RESEARCH, vol. 124, no. 1,
December 1983, pages 123-133, Elsevier Science
Publishers B.V., Amsterdam, NL; M. O'NEILL et
al.: "Structure of the acidic extracellular gelling
polysaccharide produced by Pseudomonas
elodea"

(73) Proprietor: **MERCK & CO. INC.**
126, East Lincoln Avenue P.O. Box 2000
Rahway New Jersey 07065-0900 (US)

(72) Inventor: **Peik, Jerry A.**
11291 Tribuna Avenue
San Diego California 92131 (US)
Inventor: **Steenbergen, Suzanna M.**
3105 Victoria Drive P.O. Box 636
Alpine California 92001 (US)
Inventor: **Veeder,George T.**
4494 Pocahontas Avenue
San Diego California 92117 (US)

(74) Representative: **Crampton, Keith John Allen**
et al
D YOUNG & CO 10 Staple Inn
London WC1V 7RD (GB)

EP 0 209 277 B1

Courier Press, Leamington Spa, England.

## Description

This invention relates to microbial polysaccharides. It is known that a common feature of certain microorganisms is the production of exocellular heteropolysaccharides. Heteropolysaccharides are generally linear carbohydrate polymers of high molecular weight and contain two or more kinds of monosaccharides that form a repeating unit that is polymerized.

The usefulness of most heteropolysaccharides is based on their ability to alter the viscosity and rheology of aqueous solutions. In addition, heteropolysaccharides have related secondary functions, such as emulsification, suspension, stabilization and flocculation. See for example, U.S. Patents US—A— 4,326,052 and 4,401,760.

Heteropolysaccharides are widely used in food, well-drilling, agriculture and a wide variety of other industrial applications. Commercial demand for these water-soluble gums has greatly increased over the last few decades. Furthermore, new industrial techniques create a need for heteropolysaccharides with new physical properties. Consequently, the need for heteropolysaccharides with different functionality ranges, coupled with commercial demand, has clearly indicated the necessity for the development of new heteropolysaccharides with new and different physical properties.

The present invention is based on the discovery that a novel heteropolysaccharide, herein called "Heteropolysaccharide S-657", which is composed principally of carbohydrate, about 12% protein and about 7% (calculated as O-acetyl) acyl groups, the carbohydrate portion containing about 19% by weight glucuronic acid and the neutral sugars rhamnose and glucose in the approximate molar ratio of 2:1, is produced by the action of a new strain of *Xanthomonas compestris* on a selected carbon source. This novel compound, which constitutes one embodiment of the present invention, is prepared by aerobic fermentation of a suitable aqueous nutrient medium with the new strain of *Xanthomonas campestris*. A deposit under the Budapest Treaty of a biologically pure culture of this organism was made with the American Type Culture Collection, Rockville, Maryland, on June 19, 1985 under Accession No. ATCC 53159. The novel microorganism and the production of Heteropolysaccharide S-657 by giving it in an aqueous nutrient medium by aerobic fermentation of an assimilable carbon source and recovering the S-657, constitute other embodiments of the present invention.

Heteropolysaccharide S-657 has desirable properties in aqueous systems and is especially useful in formulating oil well treating fluids. The present invention provides a well-treating fluid comprising water and from 0.01% to 1.0% by weight of heteropolysaccharide S-657.

The novel organism of the present invention was isolated from an algal sample taken from a marsh near Eureka, California. The organism was picked as a gummy colony from a YM agar plate after 4 days of incubation at 30°C. The isolate was then pure-cultured on nutrient agar.

A flask seed was started from a nutrient agar culture of the isolate. This seed was then used to inoculate another flask containing a nutrient medium having hydrolysed starch as the carbon source. After incubation, this flask was noted to contain a viscous beer and upon addition of isopropyl alcohol a fibrous material was precipitated. Another flask seed was started and used to determine the effect of various nutrient media on gum production and to determine the best growth media and fermentation conditions for this microorganism.

Heteropolysaccharide S-657 is produced during the aerobic fermentation of suitable aqueous nutrient media under controlled conditions by inoculation with a culture of the organism ATCC 53159. The media contain sources of assimilable carbon and nitrogen, and inorganic salts.

In general, carbohydrates (for example, glucose, fructose, maltose, sucrose, xylose and lactose) can be used either alone or in combination as sources of assimilable carbon in the nutrient medium. The exact quantity of the carbohydrate source or sources required in the medium depends in part upon the other ingredients of the medium, but it usually lies in the range from 2% to 5% by weight of the medium. These carbon sources may be used individually or combined in the medium.

Generally, many proteinaceous materials may be used as nitrogen sources for the fermentation process. Suitable nitrogen sources include, for example, yeast hydrolysates, primary yeast, soybean meal, cottonseed flour, hydrolysates of casein, cornsteep liquor, distiller's solubles and tomato paste. The sources of nitrogen, either alone or in combination, are used in amounts preferably ranging from 0.05% to 0.2% by weight of the aqueous medium.

Among the nutrient inorganic salts that can be incorporated in the culture media are the customary salts capable of yielding ions such as those of sodium, potassium, ammonium, calcium, phosphate, sulphate, chloride and carbonate. Also included are trace metals such as cobalt, manganese, iron and magnesium.

The nutrient media described herein are illustrative of and do not limit the wide variety of suitable media.

In the description of the fermentation process and the characterization of the microorganism, the words "Brookfield", "Fann", "Tween", "Promosoy", "Difco", "Dowex" and "Sag" are registered Trade Marks.

The Examples given are illustrative.

As an alternative medium, S-657 may be grown under low calcium ion conditions, i.e., in deionized

2

water or some other aqueous system substantially free of calcium ions (i.e., less than 4 ppm Ca$^{++}$ per 1% gum in the final fermentor broth).

The fermentation is carried out at temperatures ranging from 25°C to 35°C; optimum results being obtained at from 28°C to 32°C. The pH of the nutrient media for growing the ATCC 53159 culture and producing the heteropolysaccharide S-657 can vary from 6 to 8.

Although S-657 is produced by both surface and submerged culture, it is preferred to carry out the fermentation in the submerged state.

A small-scale fermentation is conveniently carried out by inoculating a suitable nutrient medium with the culture and, after transfer to a production medium, permitting the fermentation to proceed at a constant temperature of about 30°C on a shaker for several days.

The fermentation is initiated in a sterilized flask of medium via one or more stages of seed development. The nutrient medium for the seed stage may be any suitable combination of carbon and nitrogen sources. The seed flask is shaken in a constant temperature chamber at about 30°C for 1—2 days, or until growth is satisfactory, and some of the resulting growth is used to inoculate either a second stage seed or the production medium. Intermediate stage seed flasks, when used, are developed in essentially the same manner; that is, part of the contents of the flask from the last seed stage are used to inoculate the production medium. The inoculated flasks are shaken at a constant temperature for several days, and at the end of the incubation period the contents of the flasks are recovered by precipitation with a suitable alcohol such as isopropanol.

For large scale work, it is preferable to conduct the fermentation in suitable tanks provided with an agitator and a means of aerating the fermentation medium. According to this method, the nutrient medium is made up in the tank and sterilized by heating at temperatures of up to about 121°C. Upon cooling, the sterilized medium is inoculated with a previously grown seed of the producing culture, and the fermentation is permitted to proceed for a period of time as, for example, from 2 to 4 days while agitating and/or aerating the nutrient medium and maintaining the temperature at about 30°C. This method of producing S-657 is particularly suited for the preparation of large quantities.

Heteropolysaccharide S-657

The heteropolysaccharide produced by ATCC 53159 is composed principally of carbohydrate, about 12% protein and about 7% by weight (calculated as O-acetyl) acyl groups, and substantially no pyruvate. The carbohydrate portion of S-657 contains about 19% glucuronic acid (based on weight of gum) and the neutral sugars rhamnose and glucose in the approximate molar ratio of 2:1.

The acetyl content of about 7% was determined by two separate techniques. A 0.2% aqueous solution of S-657 gum was treated with an alkaline hydroxylamine, reagent followed by treatment with an acidic ferric chloride reagent and colorimetric analysis [See S. Hestrin (1949) *J. Biol. Chem. 180*, 249—261]. The O-acyl group was identified as O-acetyl and determined by liquid chromatography.

The neutral sugars of S-657 were also determined by various techniques. One method involved hydrolysing 50 mg of S-657 in 1 ml of 1M H$_2$SO$_4$ at 100°C for 4 hours. After cooling, 0.5 ml of 3 mg/ml xylose was added as an internal standard. Samples were neutralized by adding 3 ml of saturated Ba(OH)$_2$, then two drops of Congo Red and Ba(OH)$_2$ until the color changed to red. After centrifuging (20 minutes at 3000 RPM) the supernatants of all samples were evaporated. Dry samples were dissolved in 0.1 ml of hydroxylamine hydrochloride (40 mg/ml in dry pyridine) and heated at 90°C for 45 minutes. After cooling 0.1 ml acetic anhydride was added and the samples again heated at 90°C for 45 minutes. The sugars were separated by gas-liquid chromatography of their aldononitrile acetate derivatives and were identified and quantified by comparison with authentic standards [J. K. Baird, M. J. Holroyde, and D. C. Ellwood (1973) *Carbohydr. Res. 27*, 464—467].

The various neutral sugars of this polysaccharide were also characterized by a second method which involves dissolving approximately 2 mg of S-657 in 0.5*M* trifluoroacetic acid (2 ml). The sample was kept at 100°C overnight, concentrated to dryness and dissolve in water (2 ml). Sodium borohydride (25 mg) was added and after 2 hours the solution was treated with Dowex 50 (H$^+$) after which the pH dropped to 3.5. After filtration, the solution was concentrated and codistilled with methanol (3×5 ml). The residue was dissolved in a mixture of acetic anhydride (1 ml) and pyridine (1 ml), kept at 100°C for 1 hour and concentrated. After codistillation with toluene (3×5 ml), the residue was dissolved in methylene chloride and analysed by gas-liquid chromatography.

TABLE 1
Total neutral sugars in S-657

|  | Mole percent rhamnose | Mole percent glucose |
|---|---|---|
| Method 1 (3 samples) | 57 | 43 |
| Method 2 (9 samples) | 64 | 36 |

3

The glycuronic acid content of the polysaccharide was determined by decarboxylation with 17% hydrochloric acid, followed by trapping the liberated carbon dioxide in standard sodium hydroxide and back tritration [B. L. Browning (1967) *Methods of Wood Chemistry 2,* 632—633]. The decarboxylation method gave values between 17.6% and 19.6% for three different samples of S-657.

Paper electrophoresis was used for the separation and identification of the uronic acids present in the neutralized acid hydrolysate described above. Aliquots of this and known uronic acid standards were applied to electrophoresis paper and electrophoresis was carried out for 2 hours in a pH 2.7 buffer. Chromatograms were air dried and stained with silver nitrate to locate the uronic acids being separated. The only confirmed uronic acid spot was found to be glucuronic acid.

The absence of pyruvate was determined by adding 1 ml of a 2 mg/ml solution of S-657 to a culture tube, and adding 1 ml of 0.2N HCl, and heating at 100°C for 4 hours. A 0.5 ml sample of hydrolysate was added to 0.1 ml of reduced diphosphopyridine nucleotide (NADH) and 2.4 ml of triethanolamine solution. The absorbance was detected on a spectrophotometer and pyruvate measured. [Duckworth and Yaphe *Chem. & Ind.* (1970) p. 747]. No significant pyruvate was detected.

Nitrogen analysis was performed by Kjeldahl digestion and was determined as about 1.5% by weight nitrogen (between about 1.3% and 1.9% for 3 samples). Solids and ash analysis showed S-657 contained about 94% by weight solids (91.8%—95.8%) and 9% (7.8—10.3%) by weight ash. The protein content was determined to be about 12% (7.5%—14.5%) by the method of Lowry *et al.,* [*J. Biol. Chem.,* (1951), 193, p. 256], using bovine serum albumin as standard.

Methylation analysis was performed on partially purified samples of S-657 after dialysis and freeze drying. The samples were methylated according to the procedures outlined in Sandford & Conrad, (1966) *Biochem. 5* 1508—1507. The O-methyl ether derivatives of the sugars as their aditol acetates were separated by gas chromatography and identified by computer matching with authentic standards. The major methylated sugars identified are shown in Table 2, below.

TABLE 2
O-methyl sugars in hydrolysate
of methylated S-657

| Methylated sugar | Linkage |
|---|---|
| 2,3,4 Me$_3$ rhamnose | 1 |
| 2,3 Me$_2$ rhamnose | 1,4 |
| 2,4 Me$_2$ glucose | 1,3,6 |
| 2,6 Me$_2$ glucose | 1,3,4 |

It is to be understood that, although the methods of analysis of the heteropolysaccharide described herein were the actual methods used in arriving at the composition described above, other methods of analysis are available. Such methods of analysis should result in the same characterization of the heteropolysaccharide: however, slightly different quantitative results may be reported.

Heteropolysaccharide S-657 has been found to have outstanding properties in aqueous solution, especially in having very high viscosity at very low concentrations, good temperature stability and good foam stability. Because of this, it is useful as a thickening, suspending, emulsifying, stabilizing, lubricating, film-forming, or binding agent. S-657 has utility in various industrial, petroleum and food applications where high viscosity and excellent thermal and foam stability are desirable. In particular it has uses in the following applications or products: adhesives, wall joint cements, water-retentive grouts and mortars, spackling compounds, can sealants, boiler compounds, latex creaming, welding-rod fluxes, braising pastes, ceramic glazes and extrusions, cleaners and polishers, toys, emulsions (latex, asphalt, silicone), silver recovery, seed coatings, spray control for pesticides or herbicides, emulsifiable concentrates and flowable pesticides and herbicides, tobacco binders, water based inks, lithographic fountain solutions, leather finishes, hydromulching and hydro-seeding, textile printing and finishing, wet-end paper additives, wet-end paper retention and formation aids, anti-slick compounds, mold-release agents, liquid resins, slurry and packaged explosives, petroleum and water-well drilling muds, petroleum workover and completion fluids, petroleum stimulation fluids, cosmetics, pharmaceutical suspensions and emulsions.

This gum also has utility in food systems such as jellies and other high sugar systems, beverages including citric acid based drinks, dairy products including ice cream and yogurt, salad dressings, dry mixes, icings, and glazes, syrups, puddings, farinaceous foods, canned and retorted foods and bakery fillings.

A particularly valuable utility is in the field of petroleum and water well treating fluids and muds. Heteropolysaccharide S-657 has been found to be particularly useful in aqueous media especially formulated for use as a well-treating fluid.

Well-treating fluids refers to a broad spectrum of media that are used to facilitate operations during the various stages of drilling and using a well, such as a gas or oil well. The term "well-treating fluids" comprises, for example, circulating drilling fluids, workover and completion fluids, coring fluids, and stimulation fluids (hydraulic fracturing and acidizing) and enhanced oil recovery fluids. Materials which may be present in such fluids include sodium chloride, potassium chloride, barium sulphate, amorphous silica, calcium carbonate, bentonite, attapulgite, sodium metasulphate, quebracho, calcium lignosulfonate, lime, calcium sulphate, calcium chloride, petroleum sulfonate, tall oil soap, crude and diesel oils, starches, biocides, and polymers such as CMC, polyacrylamides, and polyacrylates. It will be appreciated that not all of these compounds will be present in any particular fluid but, rather, that compounds will be selected by the well operator in the amount necessary for the particular task to be performed. Because of the differing underground conditions experienced during and after a well is drilled, adjustments to the well treating fluid and replacement of one fluid with another are to be expected.

S-657 has been found to exhibit properties such as high viscosity for improved suspension, heat and salt stability, shear stability, and good viscosity recovery after heating and cool-down which make it desirable as a rheology modifier in well-treating fluids.

Representative well treating fluid formulations are provided in Examples 4 and 5. These formulations are not intended to be limiting but are suggestive of a range of possible well treating fluid formulations that can be prepared with S-657. Heteropolysaccharide S-657 is usable in such formulations in the range of 0.01% to 1.0% by weight.

The high viscosity of S-657 at low concentrations makes the heteropolysaccharide particularly useful as a viscosifier for enhanced oil recovery. S-657 is usable in such operations at concentrations ranging from 0.01% to 0.2% by weight, preferably in the range of 0.02% to 0.1%.

Heteropolysaccharide S-657 has a particular profile of solution properties that is a distinctive characteristic of this polysaccharide and which enables it to be distinguished over other heteropolysaccharides. S-657 has the following profile of properties: .

I. Rheological data
  1% STW[1] Viscosity (Brookfield, LVF)                                    6 RPM, 19500 mPa · s
                                                                            60 RPM, 2200 mPa · s
                                                                            6/60 ratio 8.9
  0.1% STW Viscosity Brookfield LVF with (UL adapter)                      6 RPM 95 mPa · s


II. Compatibility data [Compat.=Y, Non-Compat.=N]
  Milling Green (anionic) Compat., 1% Gum Conc., Visual                    Y
  Methylene Blue (cationic) Compat., 1% Gum Conc., Visual                  N
  CTAB (cationic) Compat., 0.5% Gum Conc., Visual                          N
  Cationic Latex Compat., 0.5% Gum Conc., Visual                          N
  Cationic Surfactant Compat., 0.4% Gum Conc., Visual                      N


III. Functionality data
  Seawater Viscosity [1 PPB (0.28% Gum Conc.) 3 RPM, Fann 35]             850 mPa · s
  Temperature Response Test[2] [Fann 50°C Viscometer at 100 $sec^{-1}$]
    Initial viscosity at 80°F (26.7°C)                                    86 mPa · s
    Viscosity upon reaching 300°F (149°C)                                 78 mPa · s (91%)
    Viscosity after 1 hour at 300°F (149°C)                               63 mPa · s (73%)
    Viscosity upon cooldown to 80°F (26.7°C)                              73 mPa · s (85%)
  Saturated NaCl Viscosity [1 PPB (0.28% Gum Conc.), 3 RPM,
    Fann 35]                                                              20 mPa · s
  Saturated $CaCl_2$ Viscosity [1 PPB (0.28% Gum Conc.), 3 RPM,
    Fann 35]                                                              10 mPa · s
  Shear Stability (1% Gum Conc.), 15 min. Blender, Init.
    Vis., (60 RPM)/% change                                              2200 mPa · s/+16
  Acrylic Latex Thickening [Latex Paint, Adhesives (PSA)]
    0.5% Gum Conc.
      Brookfield Viscosity, 60 RPM, mPa · a                               2250 mPa · s
    6 RPM/60 RPM                                                          6.9
  Acetic acid+Heat (80°C, 2 hrs.) 0.5% Gum Conc.
    Init. vis. at 9.6 $sec.^{-1}$/% change 2 hr. RT/% change
    2 hr. 80°C                                                            1000 mPa · s/+2/+6
  Heat only (80°C, 2 hrs.) 0.5% Gum. Conc. Init. vis.
    at 9.6 $sec.^{-1}$/% change                                          1000 mPa · s/+4

IV. Comments and observations:
  [1] Synthetic Tap Water: Deionized water containing 1000 ppm NaCl and 147 ppm $CaCl_2$ $2H_2O$.
  [2] 0.4% Polymer in seawater containing 500 ppm $Na_2SO_3$.

Description of the strain

Heteropolysaccharide S-657 may be prepared by fermentation of a suitable nutrient medium with a novel microorganism, which is a new strain of *Xanthomonas campestris*. A deposit under the Budapest Treaty of a biologically pure culture of the microorganism employed in making this heteropolysaccharide was made with the American Type Culture Collection, Rockville, Maryland, on June 19, 1985 under Accession No. ATCC 53159.

The ATCC also performed the taxonomic identification of the bacterial isolate S-657. Internal ATCC data for *Xanthomonas campestris* strains previously characterized by ATCC were consulted as well as various classifications in the published literature including Bergey's Manual of Systematic Bacteriology 1984, Vol. 1, Krug & Holt eds., Williams & Wilkins; D. W. Dye, *N. Zealand J. Med.*, *5*, p. 393—416 (1962), and M. P. Starr, *The Genus Xanthomonas in the Prokaryotes,* (1981).

When peritrichous flagella for this *Xanthomonas* microorganism were confirmed the recent literature was consulted, including Palleroni, N. in Clarke, P. H. and M. H. Richmond (1975), *Genetics and Biochemistry of Pseudomonas* pg. 5—6, Wiley & Sons; Palleroni, N. In *Bergey's Manual of Systematic Bacteriology* (1984), pg. 142, Volume 1; Hugh, R. and G. Gilardi in Lennette, Balows, Hausler and Truant, (1980), *Manual of Clinical Microbiology*, pg. 290, American Society for Microbiology; Palleroni, N., M. Doudoroff, R. Y. Stanier, R. E. Solanes and M. Mendel (1970), *Journal of General Microbiology*, Vol. *60: 215—231; Ulitzwr, S. (1975),* Arch. Microbiology, Vol. *104*: 285—288; and Shinoda, S. and Okamoto, K. (1977), *Journal of Bacteriology*, Vol. *129*: 1266—1271.

After review of the literature, it was determined that the morphological character of polar flagellation for *Xanthomonas*, which until recently was considered typical, has greatly reduced taxonomic significance because of cases of abnormal (i.e. lateral) flagellation. The organism was therefore classified as *Xanthomonas campestris* because it has the appropriate characteristics.

Typically, *Xanthomonas campestris* produces xanthan gum by pure culture fermentation processes. The carbohydrate portion of xanthan gum contains glucuronic acid and the neutral sugars glucose and mannose. Heteropolysaccharide S-657, which cannot be considered to be a xanthan gum, contains glucose and rhamnose, but not mannose. Thus, a novel strain of *Xanthomonas campestris,* which produces heteropolysaccharide S-657, is provided by ATCC 53159.

A. Characteristics of colony morphology

On nutriet agar, colonies appear in two days at 30°C with a diameter reaching about 0.5—1.0 mm in diameter. The colonies are round, entire, translucent, smooth and light yellow in pigment. With the addition of 1% glucose to nutrient agar, the colonies become mucoid, domed and shining.

B. Characteristics of cell morphology

The cell size is about 0.8—1.0×2.0—3.0 µm on nutrient agar, occurring singly in pairs and in long chains. The cells are gram-negative, actively motile rods with tapered ends. Flagella are peritrichous.

C. Physiological and biochemical characteristics

Table 3, below, presents the results of numerous biochemical and physiological tests employed in the identification of this microorganism:

TABLE 3

Biochemical and physiological test results for ATCC 53159 isolate

| | | | | |
|---|---|---|---|---|
| 4°C growth | − | | Gelatinase | W |
| 25°C growth | + | | Tween 20 hydrolysis | + |
| 30°C growth | + | | Tween 80 hydrolysis | + |
| 37°C growth | + | | Indole | − |
| 41°C growth | − | | Simmons citrate growth | − |
| Fluorescein produced | − | | Urease | − |
| Pyocyanine produced | − | | Nitrate to nitrite | − |
| Yellow non-diff. pigments | + | | Nitrate reduction | − |
| Melamin pigment produced | − | | Nitrite to nitrogen gas | − |
| pH 6.0 growth | + | | Hydrogen sulfide (TSI) | − |
| 1% NaCl growth (+) | + | | Lead acetate strip | + |
| 3% NaCl growth (−) | − | | Lysine decarboxylase | − |
| 6.5% NaCl growth | − | | Arginine (Mollers) | − |
| MacConkey agar growth | − | | Ornithine decarboxylase | − |
| Skim milk agar growth | + | | Phenylalanine deamination | − |
| Aesculin hydrolysis | + | | Lecithinase | − |
| Casein hydrolysis | − | | Phosphatase | − |
| Starch hydrolysis | + | | Catalase | + |
| Mucoid gr. on glucose agar | + | | Oxidase | − |
| 0.1% TTC growth | − | | Gluconate oxidation | − |
| 0.02% TTC growth | + | | Growth on malonate as SCS | − |
| | | | Tyrosine degradation | − |
| | | | dl-hydroxybutyrate growth | − |
| | | | PHB accumulation | − |
| | | | Deoxyribonuclease | W |
| | | | Growth on 0.05% cetrimide | − |
| | | | Growth on acetate as SCS | + |
| | | | Testosterone deg. | − |

TTC=triphenyl-tetrazolium chloride
W=weakly positive

7

### Sole carbon sources in Stanier's mineral base

| | | | | |
|---|---|---|---|---|
| L-arabinose | + | L-malate | | W |
| cellobiose | + | pelargonate | | − |
| D-fructose | W | propionate | | − |
| D-glucose | + | quinate | | − |
| lactose | + | succinate | | W |
| maltose | + | L-+-tartrate | | − |
| D-mannitol | − | valerate | | − |
| L-rhamnose | − | B-alanine | | − |
| D-ribose | − | D-A-alanine | | − |
| D-sorbitol | − | betaine | | − |
| sucrose | + | glycine | | − |
| trehalose | − | L-histidine | | − |
| D-xylose | + | DL-norleucine | | − |
| adonitol | − | L-proline | | − |
| erythritol | − | D-tryptophan | | − |
| glycerol | − | L-valine | | − |
| ethanol | − | DL-arginine | | − |
| geraniol | − | benzylamine | | − |
| i-inositol | − | butylamine | | − |
| sebacic acid | W | putrescine | | − |
| acetamide | − | mesconate | | − |
| adipate | − | DL-glycerate | | − |
| benzoate | − | L-tryptophan | | − |
| butyrate | − | | | |
| citraconate | − | | | |
| D-gluconate | − | | | |
| M-hydroxybenzoate | − | | | |
| 2-Ketogluconate | − | | | |
| DL-lactate | − | | | |

### Carbohydrate fermentation in O—F medium

| | | | |
|---|---|---|---|
| Acid from L-arabinose | + | Acid from adonitol | K |
| Acid from cellobiose | + | Acid from dulcitol | K |
| Acid from ethanol | K | Acide from D-galactose | + |
| Acid from D-fructose | + | Acid from inulin | K |
| Acid from D-glucose AO2 | + | Acid from salicin | − |
| Acid from D-glucose AnO2 | − | Acid from D-sorbitol | − |
| Alkaline pH in D-glucose | − | | |
| Acid from glycerol | K | | |
| Acid from i-inositol | K | | |
| Acid from lactose | + | | |
| Acid from maltose | + | +=acid | |
| Acid from D-mannitol | K | K=alkaline | |
| Acid from D-mannose | + | −=no change | |
| Acid from D-ribose | K | | |
| Acid from sucrose | + | | |
| Acid from trehalose | K | | |
| Acid from D-xylose | + | | |

Example 1

A YM flask seed was started from a 48-hour nutrient agar culture placed on a gyrotary shaker at 30°C. Approximately 24 hours later, this seed was used to inoculate a flask containing $E_1$ medium with 3% hydrolysed starch as the carbon source. This medium was also placed on a gyrotary shaker at 30°C. Approximately 72 hours later, this flask was noted to have viscous beer, and upon addition of 2 volumes of 99% isopropyl alcohol, a fibrous precipitate was noted.

$E_1$ medium contains 5 g of dipotassium phosphate, 0.1 g of magnesium sulphate, 0.9 g of ammonium nitrate, 0.5 g of Promosoy 100 (an enzymatic digest of soybean meal sold by Central Soya Chemurgy Division), 30 g of dextrose and 1 L of tap water. The pH of the $E_1$ medium is about 7.6—7.8.

Another YM seed flask was prepared in the above fashion and used at 24 hours to inoculate 5 flasks containing various media and these flasks were incubated at 30°C on a gyrotary shaker for about 72 hours at which time the pH, viscosity, gum yield and product viscosity were measured. The results are shown in Table 4 below.

TABLE 4
Effect of media on gum production

| Medium | Carbon source | pH | Beer vis(mPa · s) | % Gum yield | DI water prod vis(mPa · s) 1%/0.1% | 1% KCL prod vis(mPa · s) 1%/0.1% |
|---|---|---|---|---|---|---|
| $E_1$ | 3% hydrolysed starch | 6.6 | 1700 | 2.12 | ND | ND |
| $E_1(—NH_4NO_3+$ 0.19% $NaNO_3)$ | 3% hydrolysed starch | 6.6 | 345 | 1.86 | ND | ND |
| $E_1$ (containing 0.2% Promosoy 100) | 3% hydrolysed starch | 6.6 | 65 | 2.14 | ND | ND |
| $E_1$ | 3% glucose | 6.6 | 1000 | 1.10 | 560/14 | 560/13 |
| $E_1$+HoLe Salts[3] | 3% hydrolysed starch | 6.5 | 1750[2] | 1.20 | 880/33 | 960/32 |

[1] ND: Not determined
[2] Unutilized starch was hydrolysed with glucoamylase before precipitation with isopropyl alcohol
[3] HoLe salts: An aqueous solution (used at 1 ml/L of medium) comprising

| | Conc. in final medium (ppm) |
|---|---|
| $H_3BO_3$ | 0.05 $B^{+3}$ |
| $MnCl_2 \cdot 4H_2O$ | 0.5 $Mn^{+2}$ |
| $FeSO_4$ | 0.5 $Fe^{+2}$ |
| $CuCl_2$ | 0.01 $Cu^{+2}$ |
| $ZnCl_2$ | 0.02 $Zn^{+2}$ |
| $CoCl_2 \cdot 6H_2O$ | 0.01 $Co^{+2}$ |
| $Na_2MoO_4 \cdot 2H_2O$ | 0.01 $Mo^{+6}$ |
| Sodium tartrate | 1.8 |

As seen from the above results, the best growth medium is $E_1$ with 3% hydrolysed starch and HoLe salts.

Example 2
A fermentation procedure for producing large quantities of heteropolysaccharide S-657 is provided.
A 500 ml Erlenmeyer flask containing 100 ml of YM broth (Difco) was inoculated with a loopful of ATCC 51359 cells from a 48-hour nutrient agar plate. The flask was incubated for 24 hours at 30°C on a gyrotary shaker set at 400 rpm. A 1% inoculum was then made into two 500 ml Erlenmeyer flasks containing 100 ml each of seed medium. The seed medium contained:

| | |
|---|---|
| Glucose | 3% |
| $K_2HPO_4$ | 0.5% |
| $NH_4NO_3$ | 0.09% |
| $MgSO_4 \cdot 7H_2O$ | 0.01% |
| Promosoy 100 | 0.05% |
| HoLe salts | 1 ml/L |

The medium was prepared in tap water. HoLe salts are prepared by adding the following ingredients to one liter of deionized or distilled water:

| | |
|---|---|
| $H_3BO_3$ | 285 mg |
| $MnCl_2 \cdot 4H_2O$ | 1800 mg |
| $FeSO_4$ | 1360 mg |
| $CuCl_2$ | 26.9 mg |
| $ZnCl_2$ | 20.8 mg |
| $CoCl_2$ | 40.4 mg |
| $Mg_2MoO_4 \cdot 2H_2O$ | 25.2 mg |
| Sodium tartrate | 1770 mg |

These flasks were incubated at 30°C on a gyrotary shaker at 400 rpm for 24 hours at which point they were used to inoculate a 5 L fermentor vessel containing 3000 ml (final volume) of the same medium. The fermentation was controlled at 30°C and the aeration rate at 1 L/minute. The agitation was started at 400 rpm and increased thereafter to ensure good mixing. At 24 hours approximately 2.5 L of this seed were used to inoculate a 70 L fermentor containing 50 L (final volume) of the following medium:

| | |
|---|---|
| Glucose | 3.0% |
| $K_2HPO_4$ | 0.05% |
| $NH_4NO_3$ | 0.09% |
| $MgSO_4 \cdot 7H_2O$ | 0.01% |
| Promosoy 100 | 0.05% |
| HoLe salts | 1 ml/L |
| $Fe^{++}$ | 1 ppm |
| Sag 5691 (a defoaming agent supplied by Union Carbide) | 0.005% |

The temperature was maintained at 30°C and the aeration rate at 10 L/minute until 25 hours into the fermentation where it was adjusted to 20 L/minute. It remained at that rate for the remainder of the fermentation. The pH was controlled at greater than 6.0 by the addition of 40% KOH as needed using an automatic pH control system. The agitation was initially set at 300 rpm and was increased to 550 rpm at 25 hours, to 750 rpm at 48 hours and to 800 rpm at 72 hours. It remained at 800 rpm for the remainder of the fermentation. The results of this fermentation are given in Table 5 below.

TABLE 5

| Age | pH | Beer viscosity (mPa · s) | Gum yield (g/100 ml) | Residual carbon source |
|---|---|---|---|---|
| 0 hours | 7.0 | 5 | ND | 3.0% |
| 25 hours | 6.5 | 230 | 0.51 | ND |
| 48 hours | 6.2 | 960 | 0.80 | 1.27 |
| 72 hours | 6.3 | 2250 | 1.22 | 0.70 |
| 116 hours | 6.2 | 3550 | 1.64 | 0.24 |
| 141 hours | 7.1 | 4000 | 1.63 | 0.17 |

A total of 150 ml of 40% KOH was used to control the pH during the fermentation. The fermentation liquor was heated to approximately 75°C for 15 minutes and then cooled to approximately 30°C. The fermentation liquor was added to three volumes of 99% isopropanol. The polysaccharide precipitated as a fibrous material which was easily recovered using a sieve. The fibres were dried in a forced air tray drier at 60°C (140°F) for 2.5 hours before being milled to a powder.

Example 3

Taxonomic identification was accomplished by comparing certain physiological and biochemical characteristics of the isolate ATCC-53159 with characteristics typical of *Xanthomonas campestris* identified in Bergey's 1984 Manual and in the results of 28 strains previously characterized by ATCC. The results are shown in Table 6 below.

TABLE 6

| | Bergey's 1984 manual | ATCC data* | Isolate ATCC 53159 |
|---|---|---|---|
| Esculin | + | + | + |
| H$_2$S from Peptone | + | | + |
| Urease | − | − | − |
| Growth at 37°C | + | + | + |
| Acid in O—F medium: Arabinose | + | + | + |
| Glucose | + | + | + |
| Sucrose | + | + | + |
| Mannose | + | + | + |
| Galactose | + | + | + |
| Cellobiose | + | + | + |
| Fructose | + | + | + |
| Adonitol | − | − | − |
| Mannitol | − | − | − |
| Sorbitol | − | − | − |
| Dulcitol | − | − | − |
| Salicin | − | − | − |
| Inositol | − | − | − |
| Inulin | − | − | − |
| Trehalose | + | 95 | − |
| Utilization as SCS: Acetate | + | 25 | + |
| Citrate | + | + | − |
| Malate | + | + | + |
| Propionate | + | 5 | − |
| Succinate | + | + | + |
| Lactate | + | 40 | − |
| L-tartrate | − | − | − |
| Benzoate | − | − | − |

*Numbers indicated % positive.

Example 4

Sea water mud composition

Heteropolysaccharide S-657 is used in muds for oil well drilling. A formula and data for a seawater mud are as follows:

| S-657 | 1 pound (0.45 kg) |
| Seawater | 1 barrel (42 gal., 0.16 m³) |

Fann 35 viscosity data:

| Speed (RPM) | 3 | 6 | 100 | 200 | 300 | 600 |
|---|---|---|---|---|---|---|
| Dial reading (f1.0 spring) | 7.8 | 8.4 | 12.4 | 14.8 | 17.0 | 22.4 |
| Viscosity (mPa · s) | 780 | 420 | 37.2 | 22.2 | 17.0 | 11.2 |

Example 5

Fresh water bentonite mud

Another drilling formulation in which heteropolysaccharide S-657 is functional is as follows:

| S-657 | 1 pound (0.45 kg) |
| Bentonite | 7 pounds (3.18 kg) |
| Fresh water | 1 barrel (0.16 m³) |

Fann 35 viscosity data:

| Speed (RPM) | 3 | 6 | 100 | 200 | 300 | 600 |
|---|---|---|---|---|---|---|
| Viscosity (mPa · s) | 1160 | 610 | 48 | 28.2 | 21.6 | 14.5 |

Example 6

Solubility in salt containing systems

Heteropolysaccharide is soluble in various salt containing systems. One pound (0.45 kg) of S-657 is mixed with one barrel (0.16 m³) of the following aqueous components and the Fann 35 Viscosity is determined as follows:

Fann 35 viscosity (mPa · s) data:

| RPM | 3 | 6 | 100 | 200 | 300 | 600 |
|---|---|---|---|---|---|---|
| Fresh water | 1020 | 540 | 36.0 | 19.8 | 14.6 | 9.2 |
| 3% KCl | 920 | 510 | 42 | 23.7 | 17.6 | 10.4 |
| 15% NaCl | 580 | 320 | 31.2 | 19.2 | 15 | 9.9 |

**Claims**

1. Heteropolysaccharide S-657, which is obtainable by fermentation of a culture of *Xanthomonas campestris,* ATCC 53159, which contains no mannose or pyruvate in its structure, and which comprises principally carbohydrate, about 12% protein and about 7% (calculated as O-acetyl) acyl groups, the carbohydrate portion containing about 19% glucuronic acid, and the neutral sugars rhamnose and glucose in the approximate molar ratio of 2:1.

2. A biologically pure culture of the microorganism *Xanthomonas campestris,* ATCC 53159.

3. A culture comprising the microorganism *Xanthomonas campestris,* ATCC 53159, the said culture being capable of producing heteropolysaccharide S-657 in recoverable amounts by aerobic fermentation.

4. A process for producing heteropolysaccharide S-657 which comprises growing the microorganism *Xanthomonas campestris,* ATCC 53159, in an aqueous nutrient medium by aerobic fermentation of an assimilable carbon source and recovering the heteropolysaccharide S-657.

5. A process as claimed in Claim 4, in which the assimilable carbon source is a carbohydrate.

6. A process as claimed in Claim 5, in which the carbohydrate is hydrolysed starch.

7. A process as claimed in any one of Claims 4 to 6, in which the nutrient medium is substantially free of calcium ions.

13

8. A well-treating fluid comprising water and from 0.01% to 1.0% by weight of heteropolysaccharide S-657.

## Patentansprüche

1. Heteropolysaccharid S-657, welches durch Fermentation einer Kultur von Xanthomonas campestris, ATCC 53159, erhältlich ist, welches keine Mannose oder kein Pyruvat in seiner Struktur enthält, und welches sich hauptsächlich aus Kohlehydrat, etwa 12% Protein und etwa 7% Acylgruppen (berechnet als O-Acetyl) zusammensetzt, wobei der Kohlehydratanteil etwa 19% Glucuronsäure und die neutralen Zucker Rhamnose und Glucose im ungefähren Molverhältnis von 2:1 enthält.

2. Biologisch reine Kultur des Mikroorganismus Xanthomonas campestris, ATCC 53159.

3. Kultur, umfassend den Mikroorganismus Xanthomonas campestris, ATCC 53159, welche Kultur fähig ist, durch aerobe Fermentation Heteropolysaccharid S-657 in gewinnbaren Mengen zu produzieren.

4. Verfahren zur Herstellung von Heteropolysaccharid S-657, welches das Züchten des Mikroorganismus Xanthomonas campestris, ATCC 53159, in einem wässerigen Nährmedium durch aerobe Fermentation einer assimilierbaren Kohlenstoffquelle und das Gewinnen des Heteropolysaccharids S-657 umfaßt.

5. Verfahren nach Anspruch 4, worin die assimilierbare Kohlenstoffquelle ein Kohlehydrat ist.

6. Verfahren nach Anspruch 5, worin das Kohlehydrat hydrolysierte Stärke ist.

7. Verfahren nach einem der Ansprüche 4 bis 6, worin das Nährmedium im wesentlichen von Calciumionen frei ist.

8. Bohrlochbehandlungsflüssigkeit, umfassend Wasser und von 0,01 Gew.-% bis 1,0 Gew.-% Heteropolysaccharid S-657.

## Revendications

1. Hétéropolysaccharide S-657, que l'on peut obtenir par fermentation d'une culture de *Xanthomonas campestris,* ATCC 53159, qui ne contient ni mannose ni pyruvate dans sa structure, et qui comprend principalement des hydrates de carbone, environ 12% de protéine et environ 7% (calculés sous forme de O-acétyle) de groupes acyles, la fraction hydrate de carbone contenant environ 19% d'acide glucuronique, et les sels neutres rhamnose et glucose dans le rapport molaire approché de 2:1.

2. Culture biologiquement pure du microorganisme *Xanthomonas campestris* ATCC 53159.

3. Culture comprenant le microorganisme *Xanthomonas campestris,* ATCC 53159, ladite culture étant capable de produire l'hétéropolysaccharide S-657 en quantités récupérables par fermentation aérobie.

4. Procédé de production d'hétéropolysaccharide S-657 qui comprend la culture du microorganisme *Xanthomonas campestris,* ATCC 53159, dans un milieu nutritif aqueux, par fermentation aérobie d'une source de carbone assimilable et récupération de l'hétéropolysaccharide S-657.

5. Procédé selon la revendication 4, dans lequel la source de carbone assimilable est un hydrate de carbone.

6. Procédé selon la revendication 5, dans lequel l'hydrate de carbone est l'amidon hydrolysé.

7. Procédé selon l'une quelconque des revendications 4 à 6, dans lequel le milieu nutritif est essentiellement dépourvu d'ions calcium.

8. Fluide de traitement des puits contenant de l'eau et de 0,01% à 1,0% en poids d'hétéropolysaccharide S-657.